# EUROPEAN PATENT SPECIFICATION

(11) **EP 2 777 665 B1**
(45) Date of publication and mention of the grant of the patent: **18.05.2016**
(21) Application number: 14159389.7
(22) Date of filing: 13.03.2014
(51) Int. Cl.: A61F 13/15

(54) **Method of manufacturing a personal hygiene product**
Verfahren zur Herstellung eines Körperhygieneartikels
Procédé de fabrication d'un produit d'hygiène personnelle

(30) Priority: 13.03.2013 US 201313799495
(43) Date of publication of application: 17.09.2014
(73) Proprietor: Nordson Corporation, Westlake, OH 44145 (US)
(72) Inventor: Li, Julien, Atlanta, GA Georgia 30345 (US); Pais, George, Cumming, GA Georgia 30040 (US); Ramspeck, Alan, Cumming, GA Georgia 30040 (US)
(74) Representative: Eisenführ Speiser

(56) References cited:
- WO-A1-2012/159867
- US-A- 4 894 277
- US-A1- 2003 111 162
- US-B1- 6 552 095

## Description

### Technical Field

This invention relates to dispensing methods for applying foamed hot melt adhesive to various components of a disposable absorbent personal hygiene product during the manufacture of the disposable absorbent personal hygiene product.

### Background

Liquid adhesive, such as hot melt adhesive, is applied onto various components during manufacture of a disposable absorbent personal hygiene product such as diapers, adult incontinence products, and feminine hygiene products. Various dispensing systems have been developed for applying hot melt adhesive onto various components of the disposable absorbent personal hygiene product. In one example, these dispensing systems apply a laminating or bonding layer of hot melt adhesive between two flat substrates, such as a nonwoven fibrous layer and a thin polyethylene backsheet. In another example, hot melt adhesive is applied to one or more thin elastic strands and the strand(s) are then adhered to a nonwoven substrate to form an elasticized portion of the disposable absorbent personal hygiene product. Downstream of the dispensing system, the various components (e.g., flat substrate layers and elastic strands) pass through a pressure nip to secure the components together.

When evaluating the effectiveness of an adhesive bond between one or more elastic strands and one or more flat substrates, a characteristic that is often measured is creep resistance. "Creep" of an elastic strand is defined as the movement of either end of the elastic strand from an initial location where the end is adhered to a substrate. The level of creep resistance indicates how well the ends of the elastic strand remain adhered in position with respect to a substrate adhered to the elastic strand. Because the elastic strand is adhered to the substrate(s) in a stretched condition, the elastic strand constantly applies force to the substrate and the adhesive in an attempt to return to a relaxed, non-stretched condition. This force enables an elasticized portion of a disposable absorbent personal hygiene product (e.g., leg gathers on a diaper) to remain firmly engaged with the skin surface during use of the product. If an elastic strand in a disposable absorbent personal hygiene product undergoes any significant amount of creep after assembly, at least one end of the elastic strand will effectively de-bond from the substrate and reduce the ability of the elasticized portion to remain firmly engaged with the skin surface. In this regard, a high amount of creep may lead to failure of the disposable absorbent personal hygiene product. To avoid this undesirable creep, a high quality bond must be formed by the adhesive applied to the elastic strand so that the elastic strand does not de-bond from the substrate.

One well understood method of improving the quality of an adhesive bond and thereby reducing creep is by applying additional adhesive on the substrate(s) or the elastic strands. However, applying too much adhesive to the elastic strand locks the elastic strand along its length and thereby reduces the effectiveness of the elastic material to apply force to the substrate. In other words, the elastic strand loses the ability to apply sufficient retraction force to the substrate.

Moreover, increasing the amount of adhesive used in disposable absorbent personal hygiene product manufacturing significantly increases cost and also reduces the "hand" or softness of the resulting product. Applying too much adhesive material increases the stiffness of the resulting product and may also lead to "burn through," which occurs when the adhesive material burns or melts through the adhered substrate. Especially in diaper manufacturing, the softness of the assembled product is another important measurement used to evaluate the quality of the disposable absorbent personal hygiene product. Consequently, the amount of adhesive used to adhere elastic strands to substrates should be minimized while also maintaining a high level of creep resistance, a high retraction force, and minimized burn through and stiffness. Adhesive dispensing systems should carefully control the discharged liquid adhesive to ensure accurate placement and volume of the adhesive material while also achieving a high quality bond with minimized adhesive material. This has led to the typical use of higher density liquid adhesive on elastic strands and substrates because this liquid adhesive is easier to control during dispensing.

There is a need, therefore, for a method of dispensing adhesive in the manufacture of a disposable absorbent personal hygiene product that provides improved characteristics, including creep resistance, force retraction, and softness in the resulting product, while also minimizing the use of adhesive material in the disposable absorbent personal hygiene product.

### Summary

In one embodiment of the invention, a method of manufacturing a disposable absorbent personal hygiene product uses a stretched elastic strand and a first flat substrate portion. The method includes moving the stretched elastic strand in a machine direction past a contact nozzle. A pressurized gas and hot melt adhesive are mixed to form a foamed adhesive, which is then contact dispensed at the contact nozzle by contacting the stretched elastic strand with the foamed adhesive. Therefore, the foamed adhesive expands in volume during and after contact dispensing onto the stretched elastic strand. The method also includes securing the stretched elastic strand to the first flat substrate portion with the foamed adhesive.

In one aspect, the method includes mixing sufficient quantities of pressurized gas and hot melt adhesive to result in at least 14% total expansion in volume of the foamed adhesive deposited onto the stretched elastic strand. More particularly, the mixing of the pressurized gas and the hot melt adhesive may include sufficient quantities of pressurized gas to result in at least 26% total expansion in volume of the foamed adhesive deposited onto the stretched elastic strand, and preferably enough to result in at least 34% total expansion in volume of the foamed adhesive. This expansion provides a desirable creep resistance, such as less than 10% creep, for industry-standard add on weights.

A second flat substrate portion may also be secured to the stretched elastic strand with the foamed adhesive, thereby sandwiching the stretched elastic strand between two layers of typically nonwoven substrate material. The first and second flat substrate portions may be provided as separate substrates in some embodiments, and may alternatively be provided as separate portions of a single substrate (e.g., folded over itself) in other embodiments. In embodiments where the disposable absorbent personal hygiene product includes a plurality of stretched elastic strands, the method includes contact dispensing the foamed adhesive onto the plurality of stretched elastic strands and then securing the plurality of stretched elastic strands onto the first flat substrate portion.

In another aspect, contact dispensing the foamed adhesive further includes supplying the foamed adhesive to the contact nozzle in a discontinuous manner. This results in the foamed adhesive being discharged in a discontinuous manner onto the stretched elastic strand, and the foamed adhesive then forms localized masses configured to become discrete bond points when securing the stretched elastic strand to the first flat substrate portion. More specifically, a supply of the foamed adhesive to the contact nozzle may be turned on and off repeatedly to produce coating portions on the stretched elastic strand separated by void portions having less foamed adhesive than the coating portions. In some embodiments, this control may be used to contact dispense a higher volume per unit length of foamed adhesive onto first and third portions of a stretched elastic strand than onto a second portion of the stretched elastic strand between the first and third portions. The higher volume per unit length will therefore be located at the ends of the stretched elastic strand after securing to the first flat substrate portion, which is where a stronger bond is more important in construction of personal hygiene products such as diapers. It will be understood that the foamed adhesive may alternatively be supplied to the contact nozzle in a continuous manner, but the coating formed on the elastic strand with the foamed adhesive is believed to remain discontinuous in localized areas, which results in improved creep resistance.

The amount of pressurized gas mixed with a predetermined volume of hot melt adhesive may be increased to increase the amount of foaming that the foamed adhesive will undergo following contact dispensing at the contact nozzle. This increased foaming leads to increased creep resistance of the stretched elastic strand following securing to the first flat substrate portion. In some embodiments, the foamed adhesive is spread around on a periphery of the stretched elastic strand by wiping the foamed adhesive with a notch that the stretched elastic strand passes through at the contact nozzle. The foamed adhesive may be further spread around on the periphery of the stretched elastic strand by discharging air at the foamed adhesive at a location downstream from the contact nozzle in the machine direction. In other embodiments, the foamed adhesive may be supplied through a converging slit to increase or maintain the pressure of the foamed adhesive to limit foaming prior to contact dispensing onto the stretched elastic strand. It will be understood that contact dispensing the foamed adhesive onto the stretched elastic strand can be performed by any type of nozzle capable of contact dispensing without departing from the scope of the invention.

Various additional features and advantages of the invention will become more apparent to those of ordinary skill in the art upon review of the following detailed description of the illustrative embodiments taken in conjunction with the accompanying drawings.

### Brief Description of the Drawings

FIG. 1 is a schematic perspective view of a disposable absorbent personal hygiene product during assembly of various components.
FIG. 2 is a perspective view of foamed adhesive being contact dispensed onto an elastic strand.
FIG. 3 is a schematic diagram view of one embodiment of an adhesive dispensing system used to perform the method of the invention.
FIG. 4 is a cross-sectional side view of an exemplary foaming mixer used in the adhesive dispensing system of FIG. 3.
FIG. 5 is a schematic side view of an exemplary adhesive applicator used in the adhesive dispensing system of FIG. 3, the applicator having a contact nozzle used to produce a pattern of foamed adhesive on elastic strands as shown in FIG. 2.
FIG. 6 is a rear side view of the contact nozzle of FIG. 5, showing multiple air passages in phantom.
FIG. 7 is a bottom view of the contact nozzle of FIG. 6, further illustrating the notch and an adhesive orifice communicating with the notch.
FIG. 8 is a side cross-sectional view of the contact nozzle of FIG. 5, with adhesive material being contact dispensed onto a strand within the notch.
FIG. 9 is a detailed side cross-sectional view of the contact nozzle of FIG. 8, further illustrating the adhesive release edge of the contact nozzle.
FIG. 10 is a schematic view of another exemplary adhesive applicator used in the adhesive dispensing system of FIG. 3, the applicator having a slot nozzle used to produce a pattern of foamed adhesive on elastic strands as shown in FIG. 2.
FIG. 11 is a perspective view of the slot nozzle of FIG. 10 serving to apply adhesive to elastic strands.
FIG. 12 is an exploded perspective view of the slot nozzle of FIG. 11.
FIG. 13 is a cross-sectional view taken along the center of the slot nozzle of FIG. 11.
FIG. 14 is a graphical representation showing test results for creep resistance and add-on weight for various levels of foaming the adhesive when using the adhesive dispensing system of FIG. 3.

### Detailed Description of the Illustrative Embodiments

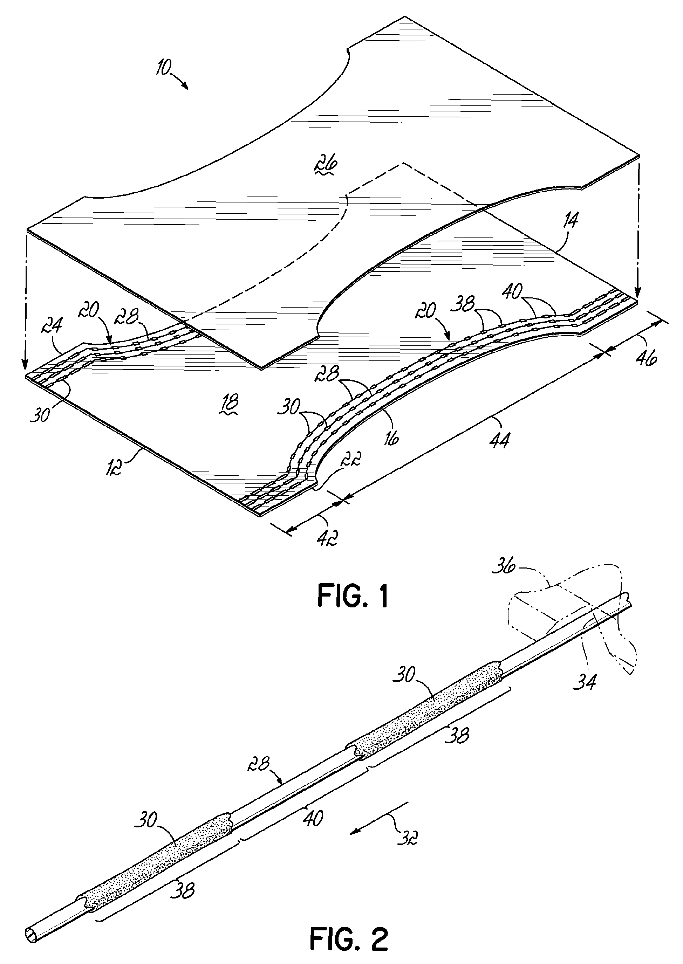

FIG. 1 illustrates one embodiment of a disposable absorbent personal hygiene product 10 manufactured using an illustrative method of the invention. The illustrated disposable absorbent personal hygiene product 10 is a disposable diaper 10 including first and second ends 12, 14 configured to wrap around the waist of the user and a narrowed central portion 16 configured to extend between the legs of the user. The diaper 10 includes a flat substrate portion 18 (hereinafter referred to as a nonwoven substrate portion 18 although other materials may be used), leg gathers 20 formed along each longitudinal side 22, 24 of the diaper 10 between the first and second ends 12, 14, and (optionally) a second flat substrate portion 26 secured to the nonwoven substrate portion 18 to enclose the leg gathers 20. The leg gathers 20 are formed by one or more elastic strands 28 that are secured to the nonwoven substrate portion 18 in a stretched condition so as to provide the diaper 10 with elasticity around the legs of the user. Although the elastic strands 28 are shown in a curved condition at the leg gathers 28, it will be understood that the elastic strands 28 may also be straight along the entire length of the diaper 10 in other embodiments. As shown, the second flat substrate portion 26 is a separate substrate from the nonwoven substrate portion 18 in the illustrated embodiment. However, it will be understood that the second flat substrate portion 26 alternatively could be a folded-over portion (not shown) of the nonwoven substrate portion 18 in other embodiments. The nonwoven substrate portion 18, leg gathers 20, and second substrate portion 26 are secured to each other with hot melt adhesive 30.

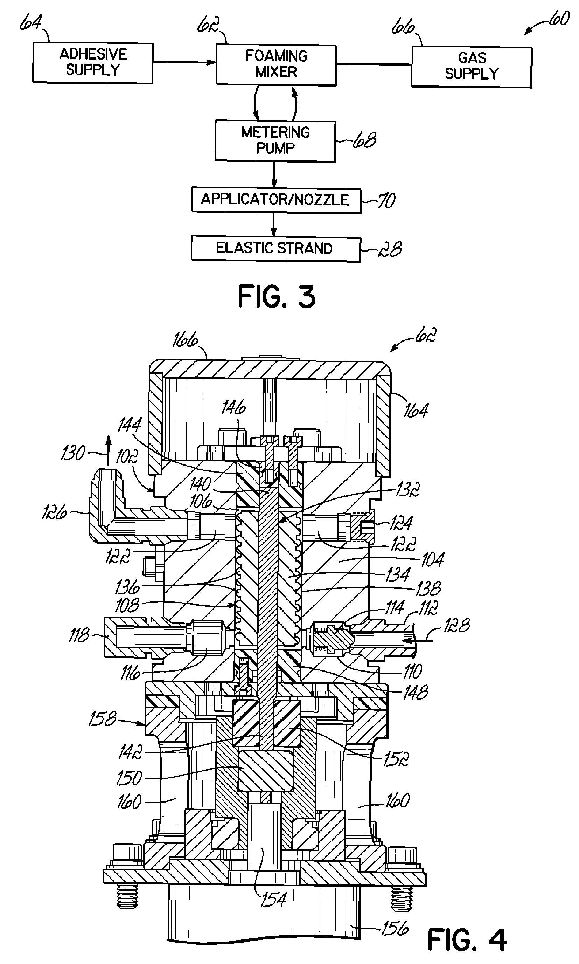

To achieve various benefits in the manufacture and use of the diaper 10, the hot melt adhesive 30 is injected with a pressurized gas such as nitrogen to form a foamed adhesive 30, which is then contact dispensed onto an elastic strand 28. Exemplary deposits of foamed adhesive 30 are shown in FIG. 1 on a plurality of elastic strands 28, and in FIG. 2 on an individual elastic strand 28. Various examples of an adhesive dispensing system configured to produce these exemplary deposits of foamed adhesive 30 are provided in FIGS. 3 through 13 and described in further detail below.

With continued reference to FIGS. 1 and 2, the foamed adhesive 30 is contact dispensed onto each of the stretched elastic strands 28 in a generally discontinuous manner so that the bond formed with the foamed adhesive 30 exhibits improved creep resistance and force retraction qualities. More particularly, each stretched elastic strand 28 moves in a machine direction shown by arrow 32 through a slot 34 of a contact nozzle 36 (shown in phantom in FIG. 2), in which a bead or coating of foamed adhesive 30 is contact dispensed onto the elastic strand 28 at an adhesive outlet (not shown). The foamed adhesive 30 is supplied in a discontinuous or on-and-off manner from the adhesive outlet such that the foamed adhesive 30 is contact dispensed in coating segments 38 separated by void segments 40 with little to no adhesive coating. It will be understood that the coating segments 38 and the void segments 40 (each shown to be roughly discrete in FIGS. 1 and 2) may blend at the junctions between these segments 38, 40 as a result of the elastic strand 28 drawing small amounts of the foamed adhesive 30 from the adhesive outlet or from the slot 34, even when adhesive delivery is temporarily stopped at the adhesive outlet. To this end, the patterns of adhesive 30 shown in FIGS. 1 and 2 are merely a schematic artist's renderings of these patterns, as it will be understood that these patterns may vary in appearance significantly in a practical setting.

The foamed adhesive 30 is spread around on the periphery of the elastic strand 28 as a result of wiping action within the slot 34 of the contact nozzle 36 and/or as a result of being impacted by process air as described in further detail below. The foamed adhesive 30 is also accelerated and stretched out when contact dispensed onto the rapidly-moving elastic strand 28, which may assist with the formation of a plurality of localized areas of increased adhesive located at or within the coating segments 38. These localized areas of adhesive at the coating segments 38 become discrete bond points spaced apart by the void segments 40 during bonding of the elastic strand 28 to the substrate portion 18, which advantageously provides desirable force retraction and creep resistance qualities. In this regard, the void segments 40 enable the stretched elastic strand 28 to contract between the coating segments 38 to thereby provide the elasticity and force retraction in the diaper 10.

Moreover, the foamed adhesive 30 may also be advantageously contact dispensed in varying total amounts per unit length at different portions of the diaper 10 when the elastic strand 28 is adhered to the flat nonwoven substrate portion 18, as described in co-owned U.S. Patent Application No. 13/444,126 to Harris. To this end, each elastic strand 28 includes a first portion 42 adjacent the first end 12 of the diaper 10, a second portion 44 extending from the first portion 42 and adjacent the central portion 16, and a third portion 46 located adjacent to the second portion 44 and the second end 14. To this end, the first and third portions 42, 46 define opposing ends of the elastic strand 28 when adhered to the nonwoven substrate portion 18, while the second portion 44 defines a central portion of the elastic strand 28 during adherence. The first portion 42 is coated with a first volume of the adhesive 30, the second portion 44 is coated with a second volume of the adhesive 30, and the third portion 46 is coated with a third volume of the adhesive 30. The second volume of the adhesive 30 is less than each of the first and third volumes of the adhesive 30. Thus, the average volume of adhesive 30 per unit length applied to the first and third portions 42, 46 of the elastic strand 28 is higher than the average volume of adhesive 30 per unit length applied to the second portion 44 of the elastic strand 28 (as schematically evidenced by the shorter void segments 40 located in the second portion 44 shown in FIG. 1). This arrangement of more volume per unit length at the ends of the elastic strand 28 provides increased foamed adhesive 30 where bond strength is most important and further reduces adhesive use in the central portion 16 of the diaper 10 where bond strength for the elastic strands 28 is less important than at the ends. The reduction of adhesive use could be up to 50% or more depending on the particular application of foamed adhesive 30 in this manner.

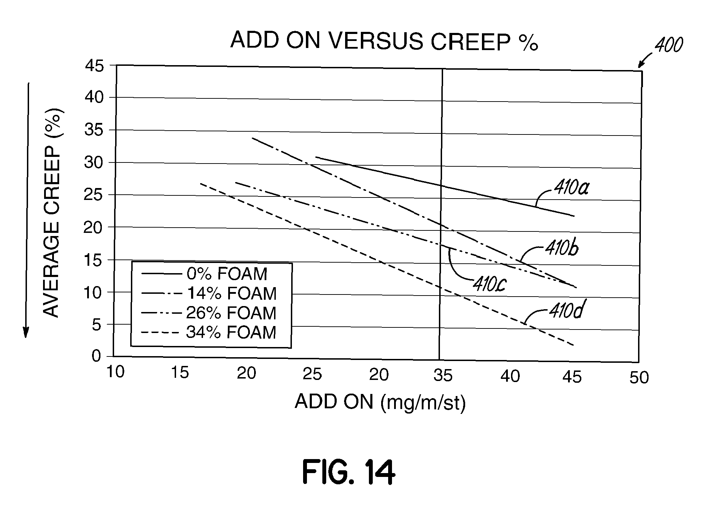

The amount of adhesive material used during construction of the elasticized portion of the diaper 10 is also reduced significantly by the foaming of the adhesive 30. The foamed adhesive 30 begins expanding in volume immediately upon exit from the adhesive outlet. The expansion in volume of the foamed adhesive 30 may occur both before and after contact dispensing of the foamed adhesive 30 on the elastic strand 28. The volume of the foamed adhesive 30 increases by up to 100% after contact dispensing, which results from the expansion of nitrogen within the hot melt adhesive 30. In this regard, the foamed adhesive 30 is up to 50% less dense than a liquid bead of the same adhesive having a similar volume. In addition to reducing the total volume of adhesive 30 being contact dispensed onto the elastic strand 28, the foamed adhesive 30 exhibits improved creep resistance compared to liquid adhesive as evidenced in test results discussed with reference to FIG. 14 below. Accordingly, the dispensing system and methods of the current application significantly reduce adhesive add on and manufacturing costs while improving and/or maintaining the bond quality necessary in the disposable absorbent personal hygiene product field.

As briefly discussed above, FIG. 3 shows an exemplary embodiment of an adhesive dispensing system 60 used to contact dispense foamed adhesive 30 onto elastic strands 28. The adhesive dispensing system 60 may include components such as the Allegro™ elastic attachment nozzles commercially available from Nordson Corporation of Westlake, Ohio, but it will be understood that any type of contact nozzle may be used without departing from the scope of the invention. The adhesive dispensing system 60 includes a foaming mixer 62 connected to an adhesive supply 64 and a pressurized gas supply 66. The foaming mixer 62 is operable to mix or inject pressurized gas into hot melt adhesive to form the foamed adhesive 30. The foaming mixer 62 is coupled to a metering pump 68, such as a gear pump, and to an adhesive contact dispenser or applicator 70. The metering pump 68 supplies a metered amount of foamed adhesive 30 to the applicator 70 while recycling the remaining foamed adhesive 30 back to the foaming mixer 62. Thus, the foamed adhesive 30 in the metering pump 68 remains pressurized and continuously replenished by the foaming mixer 62. The applicator 70 contact dispenses the foamed adhesive 30 and discharges the process air to produce a desired deposit of foamed adhesive 30 on an elastic strand 28 as described above with reference to FIGS. 1 and 2.

In one embodiment, the foaming mixer 62 is a mixer as described in U.S. Patent No. 7,703,705 to Ganzer. This exemplary embodiment of a foaming mixer 62 is also shown in FIG. 4. The foaming mixer 62 includes a mixing body 102 with a side wall 104, a tubular mixing chamber 106 bound by the side wall 104, and a mixer element 108 located inside the mixing chamber 106. The mixer 62 is heated by heaters (not shown), such as cartridge-style resistance heating elements, embedded in the side wall 104. The heaters are controlled using feedback from a conventional temperature sensor (not shown), such as a resistance temperature detector, a thermistor or a thermocouple. The heaters ensure that the adhesive material in the mixer 62 is maintained within an acceptable temperature range for dispensing by the applicator 70.

In one example, the foaming mixer 62 receives Bostik 2861 hot melt adhesive and nitrogen supplied at about 900 psi. The foaming mixer 62 is maintained at about 315 degrees Fahrenheit and operates at about 240 rpm such that the foamed adhesive 30 exits the foaming mixer 62 at a pressure of about 900 psi. With these settings, the foaming mixer 62 is operative to discharge about 20-45 milligrams per meter of elastic strand 28 when the adhesive dispensing system 70 contact dispenses adhesive onto an elastic strand 28.

The foaming mixer 62 includes an adhesive inlet port 110 leading into the mixing chamber 106. The adhesive supply 64 is coupled in fluid communication with the adhesive inlet port 110 by an elbow fitting 112 mounted in the adhesive inlet port 110 by, for example, a threaded engagement and a supply hose (not shown) connected to the elbow fitting 112. A flow control element such as a spring-loaded check valve 114 is located in the adhesive inlet port 110 between the mixing chamber 106 and the adhesive supply 64. The check valve 114, which has a conventional construction, prevents gas-filled adhesive material from infiltrating into the elbow fitting 112 and being transported upstream to the adhesive supply 64.

The foaming mixer 62 also includes a gas inlet port (not shown) leading into the mixing chamber 106. The gas supply 66 is coupled in fluid communication with the gas inlet port. The gas inlet port may be disposed adjacent to the adhesive inlet port 110 in the mixing body 102. The foaming mixer 62 also includes a measurement port 116 extending into the mixing chamber 106 opposite the adhesive inlet port 110. The measurement port 116 receives an elbow connector 118 configured to receive a pressure gage (not shown) for measuring the pressure within the mixing chamber 106. The foaming mixer 62 includes a pair of outlet ports 122 extending into the mixing chamber 106. One of the outlet ports 122 is inactive in FIG. 4 and closed with a plug 124, while the other outlet port 122 receives an elbow fitting 126 leading to the metering pump 68 and the applicator 70. Consequently, adhesive material enters the foaming mixer 62 at the adhesive inlet port 110 as shown by arrow 128 and leaves the foaming mixer 62 at the outlet port 122 as shown by arrow 130.

The mixer element 108 includes a central shaft 132 extending longitudinally through the mixing chamber 106, a cylindrical body 134 rigidly coupled for rotation with the central shaft 132, and fins 136 that project outwardly from the cylindrical body 134 toward the confronting inner surface 138 of the side wall 104 of the mixing chamber 106. The central shaft 132 includes a first end 140 located adjacent to the outlet ports 122 and a second end 142 located adjacent to the adhesive inlet port 110. The first end 140 of the central shaft 132 is supported for rotation relative to the side wall 104 by a bushing or bearing 144 in the mixing chamber 106. A thrust bearing 146 fitted in the bushing 144 provides a thrust load support for central shaft 132. The bushing 144 and the thrust bearing 146 are assembled together and secured to the mixing body 102 with conventional threaded fasteners.

The second end 142 of the central shaft 132 projects through another bushing 148 situated in the mixing chamber 106 adjacent the adhesive inlet port 110. Another thrust bearing 150 provides a thrust load support for the second end 142 of the central shaft 132. The second end 142 of the central shaft 132 is coupled by a coupling element 152 with a drive shaft 154 of a motor 156. The coupling element 152 and the thrust bearing 150 adjacent the coupling element 152 are formed from a material having a low thermal conductivity so that heat transfer is reduced from the mixing body 102 to the motor 156. The motor 156 is also isolated thermally from the mixing body 102 by a standoff 158 separating the motor 156 from the mixing body 102. The standoff 158 includes slots 160 that promote cooling at locations between the mixing body 102 and the motor 156. The motor 156 drives the powered rotation of the drive shaft 154 and the central shaft 132 for moving the fins 136 relative to the side wall 104 of the mixing chamber 106.

The adhesive material is bounded inside the mixing chamber 106 in a region between the bushings 144, 148. The bushings 144, 148 include various sealing members that assist in confining the fluid material inside the mixing chamber 106. A cowling 164 and a cap 166 are secured by conventional fasteners to the mixing body 102 and protectively cover the mixing body 102 opposite the motor 156.

The fins 136 on the mixer element 108 are distributed in rows along the length of the cylindrical body 134 (and the length of the central shaft 132). The tip of each fin 136 has a close clearance with the side wall 104. The adhesive material and the pressurized gas delivered into the mixing chamber 106 are forced through gaps between adjacent fins 136, as the fins 136 rotate, for mixing, stirring and agitating the gas and adhesive material into the foamed adhesive 30. Rotation of the fins 136 relative to the stationary side wall 104 therefore operates to repeatedly divide the gas and adhesive into small streams and then recombine the streams to create a substantially homogeneous blend or mixture of gas and adhesive with the gas in solution.

The fins 136, which are fashioned from an initially continuous helical thread extending along the length of the cylindrical body 134, define a helical arrangement likewise winding along the length of the cylindrical body 134. As the central shaft 132 of the mixer element 108 is continuously rotated by operation of motor 156, the helical arrangement of the fins 136 tends to force the adhesive material toward the adhesive inlet port 110, which counters the forward flow of the gas/adhesive mixture toward the outlet ports 122. The foaming mixer 62 is therefore operable to supply foamed adhesive 30 to the metering pump 68 and to the applicator 70. Additionally, the foaming mixer 62 is smaller in size than many conventional foaming mixers, which enables the foaming mixer 62 to be positioned in close proximity to the metering pump 68 and the applicator 70.

The metering pump 68 may be the gear pump used with the VersaBlue® melters commercially available from Nordson Corporation of Westlake, Ohio. The metering pump 68 operates at a rotational speed similar to the rotational speeds used during metering of liquid adhesive because the pressurized foamed adhesive 30 in the metering pump 68 is in substantially a liquid state. For example, the foamed adhesive 30 may circulate between the foaming mixer 62 and the metering pump 68 at about 900 psi to maintain the substantially liquid state until contact dispensing at the applicator 70. The metering pump 68 then delivers a metered supply of the foamed adhesive 30 into one of the applicators 70 described below. It will be understood that the metering pump 68 recirculates foamed material back to the foaming mixer when the applicator 70 does not require foamed adhesive 30 and also that different types of pumps may be used in other embodiments.

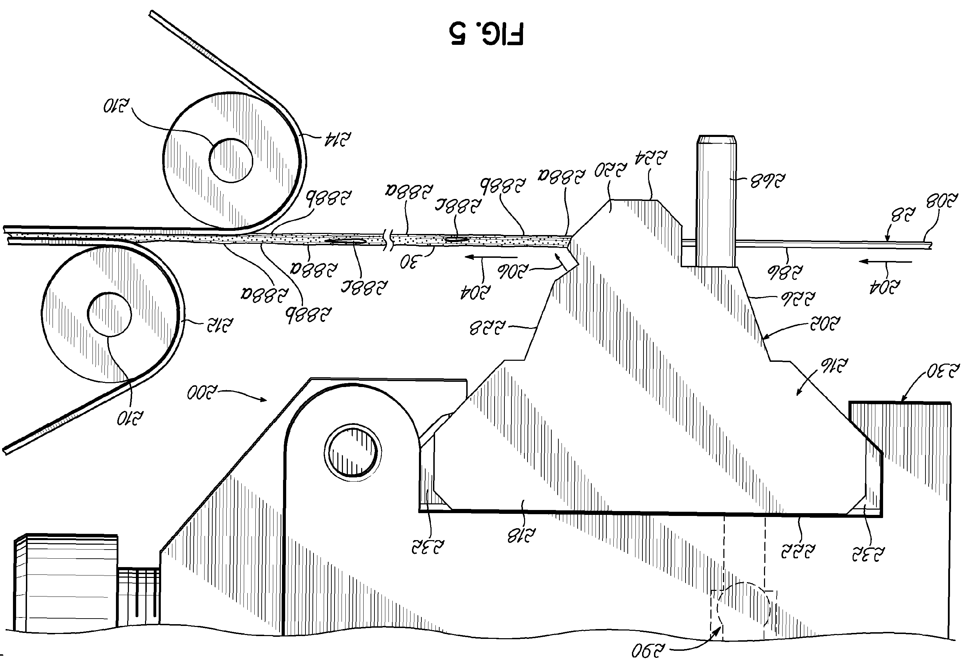

In one embodiment, the applicator 70 includes an adhesive contact dispenser 200 with a contact nozzle 202 as described in U.S. Patent Publication No. 2012/0258246 to Saine et al.,. This embodiment of a contact nozzle 202 is also shown in FIGS. 5-9. Although two exemplary embodiments of contact nozzles for performing the method of the current invention are described in detail below, any other type of nozzle capable of contact dispensing may be used in accordance with this invention.

With particular reference to FIG. 5, the contact nozzle 202 is coating one or more stretched elastic strands 28 with a foamed hot melt adhesive 30 so as to form an elasticized portion of a hygiene product 10. The contact nozzle 202 applies foamed adhesive 30 onto the stretched elastic strand 28 as the elastic strand 28 moves in a machine direction as indicated by arrows 204 in FIG. 5. The contact nozzle 202 then discharges pressurized air at the foamed adhesive 30 as shown by arrows 206 to cause the foamed adhesive 30 to further spread around on a periphery 208 of the elastic strand 28. The elastic strand 28 then continues in the machine direction to first and second bonding reels 210 that couple first and second nonwoven substrate portions 212, 214 such as top and bottom sheets of a typical diaper 10 to the elastic strand 28 in a sandwich-like construction.

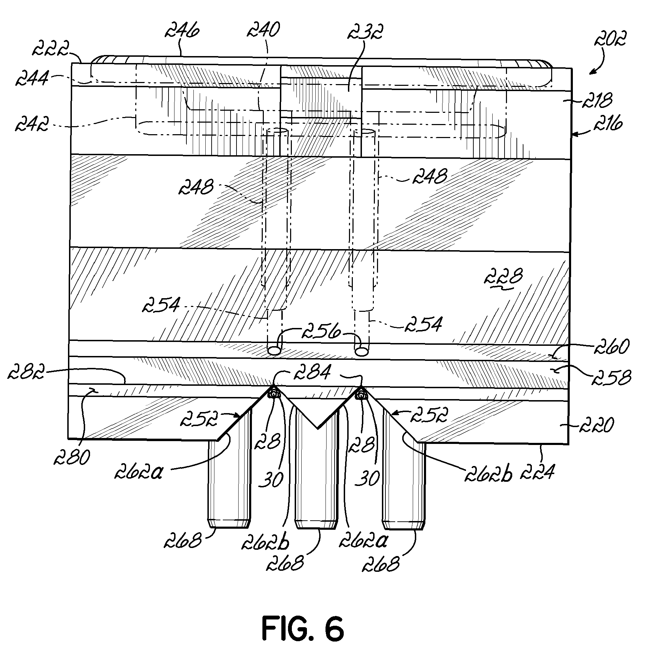

The contact nozzle 202 is shown in further detail in FIGS. 6 through 9. The contact nozzle 202 is a V-notch contact nozzle 202 including a nozzle body 216 having an upper body portion 218 and a lower body portion 220. The nozzle body 216 also includes a top side 222, a bottom side 224, a front side 226 extending between the top and bottom sides 222, 224, and a rear side 228 extending between the top and bottom sides 222, 224. The top side 222 defines a mounting surface configured to abut a module 230 when the contact nozzle 202 is coupled to the module 230. The upper body portion 218 is generally longer along the machine direction than the lower body portion 220 from the front side 226 to the rear side 228, thereby giving the contact nozzle 202 a tapered appearance from the top side 222 to the bottom side 224. Thus, the upper body portion 218 defines connection portions 232 along the front side 226 and the rear side 228 for aligning the contact nozzle 202 with the module 230. The contact nozzle 202 is clamped to the module 230 such that the top side 222 is coupled to the module 230. In some embodiments, the nozzle body 216 may have a different shape and size, including, but not limited to, being formed by stacked plates.

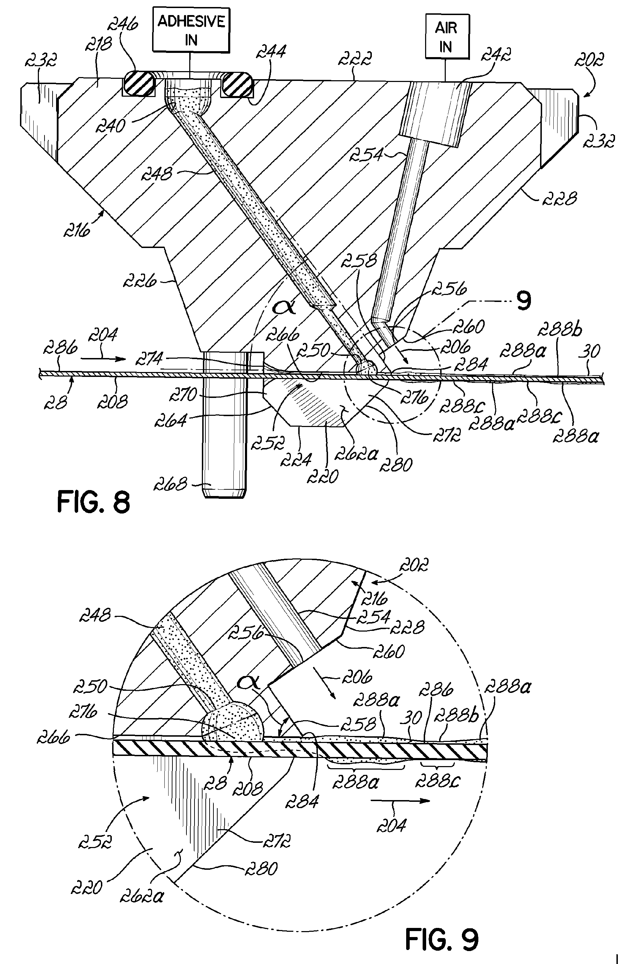

The contact nozzle 202 further includes an adhesive inlet 240 and an air inlet 242 disposed along the mounting surface at the top side 222 of the nozzle body 216. The adhesive inlet 240 is surrounded by a seal groove 244 that receives a seal member 246 between the contact nozzle 202 and the previously-described module 230. The adhesive inlet 240 is fluidically coupled to a plurality of adhesive passages 248 formed in the nozzle body 216 and extending into the lower body portion 220 of the nozzle body 216. Although two adhesive passages 248 are shown in FIGS. 6 and 8, more or fewer adhesive passages 248 may be coupled to the adhesive inlet 240 in other embodiments of the contact nozzle 202. Each adhesive passage 248 is spaced from adjacent adhesive passages 248 in a lateral direction transverse to the machine direction. Each adhesive passage 248 delivers foamed adhesive 30 from the adhesive inlet 240 to an adhesive orifice 250 communicating with a respective slot in the form of a V-shaped notch 252 (hereinafter V-notch 252) formed near the bottom side 224 of the nozzle body 216.

In a similar manner, the air inlet 242 is fluidically coupled to a plurality of air passages 254 formed in the nozzle body 216 and extending into the lower body portion 220. Each air passage 254 is positioned proximate to and directly rearward of the respective adhesive passage 248 within the nozzle body 216. In this regard, each set of one adhesive passage 248 and one air passage 254 coats one elastic strand 28 passing through the contact nozzle 202. Each air passage 254 delivers air from the air inlet 242 to an air orifice 256 directed at the foamed adhesive 30 in contact with the elastic strand 28. More particularly, the air orifice 256 is positioned adjacent to a rear surface 258, which is part of the rear side 228 of the nozzle body 216. As such, air discharged from the air passage 254 and the air orifice 256 is directed along the rear surface 258 to act on the foamed adhesive 30 as the elastic strand 28 exits the V-notch 252. The air orifice 256 is formed in an intermediate surface 260 extending from the rear surface 258.

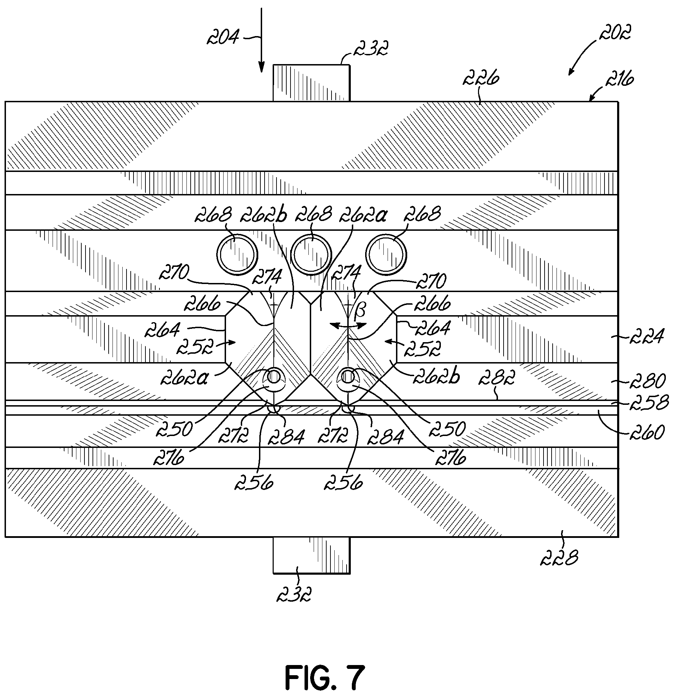

With reference to FIGS. 6 and 7, the V-notches 252 of the nozzle body 216 are shown in greater detail. In this regard, each V-notch 252 is defined by two elongate converging surfaces 262a, 262b extending from an access slot 264 defined at the bottom side 224 of the nozzle body 216 to a top edge 268 where the converging surfaces 262a, 262b intersect. Each of the converging surfaces 262a, 262b is generally planar such that the V-notch 252 defines a notch angle β between the converging surfaces 262a, 262b. The notch angle β is illustrated in this exemplary embodiment as about 90 degrees, although it will be understood that the notch angle β may alternatively be within a range of about 60 degrees to about 90 degrees in other embodiments consistent with the current invention. The access slot 264 communicates with the V-notch 252 so that an elastic strand 28 can be inserted upwardly from below the nozzle body 216 into position within the V-notch 252. More specifically, the elastic strand 28 is moved from the access slot 264 into engagement with both converging surfaces 262a, 262b adjacent the top edge 266. The top edge 266 is preferably formed so as to be dead sharp between the converging surfaces 262a, 262b, but it will be understood that the top edge 266 may define a radius of curvature of up to 0.01 inches without departing from the scope of the invention.

Although no additional strand guide element is necessary with the nozzle body 216 to position the elastic strand 28 within the V-notch 252, the contact nozzle 202 also includes a series of alignment pins 268 extending downwardly from the front side 226 of the nozzle body 216. The alignment pins 268 are therefore located a small distance upstream from the V-notches 252 in a machine direction as previously described. More specifically, each V-notch 252 includes an inlet end 270 bounded in opposing lateral directions by two of the alignment pins 268. When an elastic strand 28 is moved upwardly through the access slot 264, the elastic strand 28 is therefore also positioned between these two alignment pins 268. The alignment pins 268 function to prevent "jumping" or unintentional movement of an elastic strand 28 from one V-notch 252 to another V-notch 252. For example, an elastic strand 28 may include a knot tied between free ends of two supply reels of the elastic strand 28 in order to enable continuous running of the elastic strand 28 through the contact nozzle 202. When such a knot encounters the inlet end 270 of the V-notch 252, the larger size of the knot may cause the elastic strand 28 to "jump" temporarily away from the top edge 266 of the V-notch 252 towards the access slot 264. This jump away from the V-notch 252 may be significant enough to move the elastic strand 28 below the access slot 264, which could hypothetically lead to re-entry of that strand 28 into a different adjacent access slot 264 and V-notch 252. However, the alignment pins 268 prevent such a jump into an adjacent access slot 264 and V-notch 252 when such an event occurs. Although the alignment pins 268 define a generally cylindrical shape in the illustrated embodiment to reduce any potential frictional contact with the elastic strands 28, it will be understood that differently shaped and sized alignment pins 268 may be used in other embodiments.

Each V-notch 252 also extends to an outlet end 272 located at the rear side 228 of the nozzle body 216. Adjacent the inlet end 270, the converging surfaces 262a, 262b include chamfered opening portions 274 that broaden the size of the opening into the V-notch 252, thereby reducing a likelihood of the elastic strand 28 running past a sharp edge of the nozzle body 216. Over halfway along the length of the V-notch 252 (e.g., at a location closer to the outlet end 272 than the inlet end 270), the V-notch 252 is in fluid communication with the adhesive passage 248 via the adhesive orifice 250. An expansion chamber 276 is formed by using a ball-nose shaped mill to expand the size of the intersection between the V-notch 252 and the adhesive orifice 250. The expansion chamber 276 includes a rounded profile and extends a small distance above the top edge 266 of the V-notch 252 such that the adhesive orifice 250 defines a substantially planar outlet for adhesive material to flow into the expansion chamber 276. The addition of the expansion chamber 276 enables the use of a smaller diameter adhesive orifice 250, such as 0.020 inches in the exemplary embodiment, which reduces the likelihood of adhesive material dripping out of the adhesive orifice 250 between dispensing cycles. In one example, the adhesive orifice 250 may define a diameter of about 0.020 inches (0.051 cm) while the expansion chamber 276 defines a diameter of about (0.0635 cm) to about 0.035 inches.

As shown most clearly in FIG. 9, the rear surface 258 of the nozzle body 216 also intersects a lower rear surface 280 at an elongate edge 282. The outlet end 272 of the V-notch 252 intersects this lower rear surface 280 such that the top edge 266 intersects the elongate edge 282 at an adhesive release edge 284. The top edge 266 and the rear surface 258 define an interior angle α at the adhesive release edge 284. The interior angle α is an acute angle so that the adhesive release edge 284 promotes sharp release of the foamed adhesive 30 on the elastic strand 28 from the nozzle body 216. The interior angle α is measured in an upstream direction along the machine direction from the adhesive release edge 284. In the illustrated embodiment, the acute angle from the machine direction may be in the range of about 50 degrees to about 80 degrees. As the acute angle α is made smaller within this range, the air flow from the air orifice 256 becomes more parallel to the movement of the elastic strand 28 along the machine direction, which enables higher air pressures to be used for the air flow to further spread the foamed adhesive 30 without blowing the foamed adhesive 30 off of the elastic strand 28.

The operation of the contact nozzle 202 is shown in further detail in FIGS. 8 and 9. As described briefly above, the foamed adhesive 30 is discharged from the adhesive passage 248 through the adhesive orifice 250 and into the expansion chamber 276 adjacent the top edge 266 of the V-notch 252. The foamed adhesive 30 is contact dispensed onto an upper surface 286 of the elastic strand 28 at the expansion chamber 276, and the elastic strand 28 effectively divides at least a portion of the foamed adhesive 30 flowing out of the expansion chamber 276 to force the foamed adhesive 30 to move along the converging surfaces 262a, 262b of the V-notch 252 and begin spreading around the elastic strand 28. Because the elastic strand 28 passes the expansion chamber 276 at a greater velocity than the foamed adhesive 30 is supplied to the expansion chamber 276, the elastic strand 28 effectively draws the foamed adhesive 30 from the expansion chamber 276 in a semi-starved state and the foamed adhesive 30 does not have any opportunity to fly off the elastic strand 28. Consequently, when the elastic strand 28 reaches the outlet end 272 of the V-notch 252, the foamed adhesive 30 is already beginning to spread and move around on the periphery 208 of the elastic strand 28.

Upon release from the nozzle body 216, the foamed adhesive 30 (which is likely continuing to expand) in contact with the elastic strand 28 is struck by additional air discharged from the air orifice 256 toward the elastic strand 28. The air causes the foamed adhesive 30 to spread more around the periphery 208 of the elastic strand 28 in order to coat substantially the entire periphery 208 of the elastic strand 28 with the foamed adhesive 30. It is believed that the mechanical movement of the foamed adhesive 30 with the converging surfaces 262a, 262b immediately before this impact of the air further enhances the spreading effects caused by the air. The air discharged from the air orifice 256 does not blow the foamed adhesive 30 off of the elastic strand 28 because the foamed adhesive 30 is contact dispensed onto the elastic strand 28 and begins forming an adhesive bond with the elastic strand 28 within the V-notch 252 prior to being struck with the air.

The foamed adhesive 30 forms a coating on the elastic strand 28 that appears continuous to the naked eye, but it is believed that this coating is not entirely continuous along the length of the elastic strand 28. As described above, the foamed adhesive 30 is contact dispensed onto the elastic strand 28 in a semi-starved state, which causes the amount of foamed adhesive 30 to vary along the length of the elastic strand 28. More particularly, the foamed adhesive 30 is believed to form localized masses or thicker sections separated by thinner sections on the elastic strand 28. In this regard, FIGS. 8 and 9 schematically illustrate that the foamed adhesive 30 forms a coating with a plurality of thicker portions 288a, a plurality of thinner portions 288b, and preferably a plurality of void portions 288c where no foamed adhesive 30 is on the elastic strand 28. These portions 288a, 288b, 288c are shown as an artist's rendering and it will be appreciated that the actual appearance and distribution of these portions 288a, 288b, 288c may vary in actual use depending on operation parameters such as air pressure. The thickness irregularities of the coating believed to be formed by the foamed adhesive 30 advantageously results in the thicker portions 288a functioning as discrete bond points formed along the length of the elastic strand 28 when adhered to one or more of the substrate portions 212, 214, as described in detail above. In addition, the supply of foamed adhesive 30 to the adhesive orifice 250 may be selectively controlled in an on-off manner by a valve 290 located in the module 230, and this can also assist with the formation of the thickness irregularities as well as coating portions 38 and void portions 40 along the length of the elastic strand 28. Accordingly, the desired volume per unit length and discrete bond points shown in FIGS. 1 and 2 may be contact dispensed using the adhesive contact dispenser 200 of this embodiment.

In another similar embodiment, the contact nozzle 202 as shown in FIG. 8 may be modified to remove the air inlet 242, the air passage 254, and the air orifice 256 from the nozzle body 216. The contact nozzle 202 would continue to contact dispense the foamed adhesive 30 onto each elastic strand 28 as described in detail above, but there would be no downstream spreading using pressurized air. However, the foamed adhesive 30 may still spread around on the periphery of the elastic strand 28 as a result of the wiping action of the V-notch 252 and as a result of expansion of the foamed adhesive 30 after contact dispensing onto the elastic strand 28. To this end, the design of the contact nozzle or applicator may be changed without departing from the scope of the methods described in this invention.

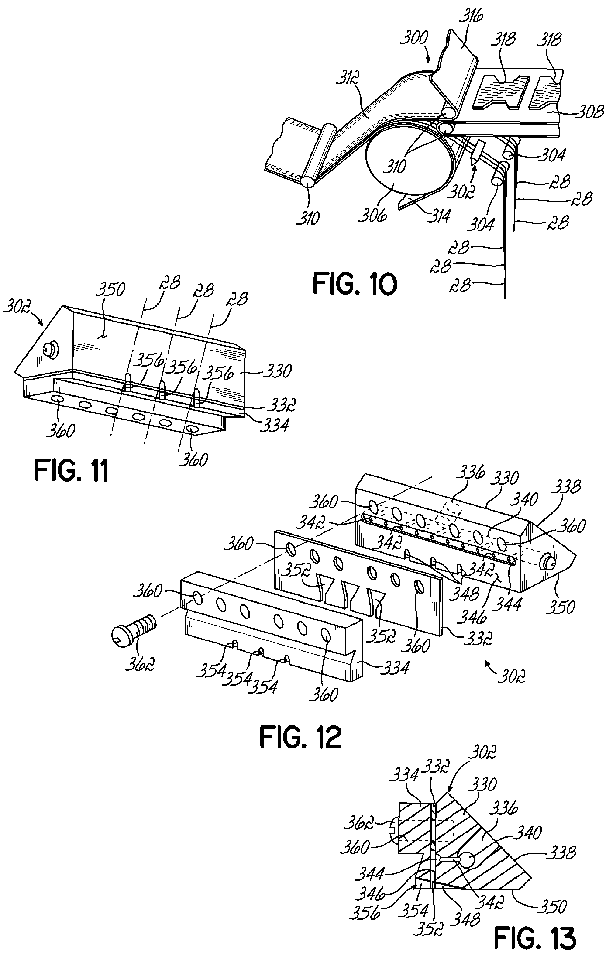

In another embodiment, the applicator 70 includes an adhesive contact dispenser 300 having a slot nozzle 302 as described in U.S. Patent No. 4,687,477 to Suzuki et al. This exemplary embodiment of a slot nozzle 302 is also shown in FIGS. 10-13.

With reference to FIGS. 10-13, the adhesive contact dispenser 300 includes guide rolls 304 configured to receive corresponding pluralities of elastic strands 28, the slot nozzle 302 located downstream from the guide rolls 304, and a chilling roll 306 located downstream from the slot nozzle 302. In addition to the chilling roll 306, the adhesive contact dispenser 300 includes a conveyor 308 and a plurality of pressure rolls 310 that are configured to transport and press various components of a continuous diaper material 312 together, including, but not limited to, the elastic strands 28, first and second flat substrate portions 314, 316 (each of which may be formed from nonwoven material), and absorbent padding 318. Therefore, the slot nozzle 302 applies foamed adhesive 30 to each of the elastic strands 28 prior to the elastic strands 28 being bonded to one or more of the other components forming the continuous diaper material 312. As is well understood in the art, the continuous diaper material 312 is transferred to a subsequent station (not shown) in which the continuous diaper material 312 will be cut into individual diapers 10 such as those shown in FIG. 1.

FIGS. 8 through 10 illustrate an embodiment of the slot nozzle 302 in further detail. The slot nozzle 302 includes a triangular prism-like first member 330, a plate-like second member 332 and a plate-like third member 334. The first member 330 includes an inlet hole 336 opening at the center of one side wall 338, and a distribution channel 340 communicating with the inlet hole 336 and extending longitudinally through a substantial portion of the first member 330. The first member 330 also includes a plurality of jet orifices 342 communicating with the distribution channel 340 and opening, at regular intervals, into an elongate recess 344 formed longitudinally in another side wall 346 opposite the side wall 338 with the inlet hole 336. A plurality of slits 348 is formed at regular intervals in a bottom wall 350 of the first member 330, the bottom wall 350 generally extending between the two side walls 338, 346. As shown in FIG. 10, a flow path is defined for foamed adhesive 30 through the first member 330, the flow path beginning at the inlet hole 336 and then extending through the distribution channel 340 and the jet orifices 342 to the elongate recess 344.

The second member 332 includes a plurality of generally triangular slits 352 at the same intervals as the slits 348 in the bottom wall 350 of the first member 330. The triangular slits 352 are captured between the sidewall 346 with the elongate recess 344 in the first member 330 and the third member 334 when the slot nozzle 302 is fully assembled. The triangular slits 352 extend from the elongate recess 344 down to the slits 348 so as to provide a communication path between the elongate recess 344 and the slits 348. In this regard, the triangular slits 352 continue the flow path defined through the first member 330 for the foamed adhesive 30.

The third member 334 also includes a plurality of slits 354 at the same intervals as the slits 348 in the first member 330 and the triangular slits 352 in the second member 332. In the fully assembled state, the plurality of slits 354 align with and are adjacent to the triangular slits 352 and the slits 348 in the first member 330 so as to collectively define strand slots 356 configured to receive individual elastic strands 28 to be coated with foamed adhesive 30. Each of the first, second, and third members 330, 332, 334 also includes threaded apertures 360 that align when the first, second, and third members 330, 332, 334 are located adjacent to one another. The members 330, 332, 334 are connected to form the complete slot nozzle 302 by a plurality of threaded screws 362 engaged with these threaded apertures 360.

In operation, an adhesive source (such as the foaming mixer 62 and metering pump 68) adapted to control or regulate supply of foamed adhesive 30 is connected with the inlet hole 336. When passing through the respective strand slots 356, the elastic strands 28 are contact dispensed with the foamed adhesive 30 supplied from the reservoir and along the flow path defined through the inlet hole 336, the distribution channel 340, the jet orifices 342, the elongate recess 344, and the triangular slits 352 into the strand slots 356. The various orifices and channels (336, 340, 342, 344, 352, 356) forming the flow path for the foamed adhesive 30 within the slot nozzle 302 may be dimensioned so that the foamed adhesive 30 remains above a critical pressure to keep substantially all of the gas entrained in solution with the liquid adhesive before contact dispensing at the strand slots 356. For example, the triangular slits 352 are shown to converge in the flow direction so as to maintain or increase the pressure of the foamed adhesive until contact dispensing at the strand slots 356. In some embodiments, a collection reservoir (not shown) may be positioned below the strand slots 356 to collect excess foamed adhesive 30; however, the foamed adhesive 30 is generally contact dispensed in a semi-starved state to the elastic strands 28 so that excess foamed adhesive 30 should not need to be collected by this collection reservoir. Similar to the previous embodiment, the supply of foamed adhesive 30 may be controlled in an on-off manner to produce the previously-described coating portions 38 and void portions 40 on each elastic strand 28, thereby providing discrete bond points in the final assembled hygiene product 10. However, in some embodiments, the supply of foamed adhesive 30 may also be generally continuous so that the foamed adhesive 30 is applied continuously to the elastic strands 28 (however, the coating formed by the foamed adhesive 30 is still believed to be discontinuous in localized positions as a result of the coating at a semi-starved state in the strand slots 356.

In both embodiments of a contact or slot nozzle 202, 302 described above, it will be understood that the number of slots or notches for receiving elastic strands 28 may be modified to fit a particular application without departing from the scope of the invention. Furthermore, the particular structure of the contact and slot nozzles 202, 302 may be modified in other embodiments as long as the method of applying foamed adhesive 30 onto the strands 28 is maintained. In this regard, the contact and slot nozzles 202, 302 and adhesive contact dispensers 200, 300 described above are merely two examples of equipment that may be used to perform the advantageous methods of the current invention. Any nozzle or applicator capable of contact dispensing may be used in other embodiments consistent with this invention.

When using the foamed adhesive 30 to bond one or more elastic strands 28 to a nonwoven substrate portion 18, the bond quality exhibited by the foamed adhesive 30 is substantially similar to or better than the bond quality formed by liquid adhesive. For example, using the same volume of foamed adhesive 30 and liquid adhesive results in substantially similar levels of creep resistance. Furthermore, the foamed adhesive 30 continues to form discrete bond points along the elastic strand 28 during bonding, which provides high force retraction qualities. Considering that the foamed adhesive 30 in such an example includes about half of the normal amount of hot melt adhesive material as a liquid adhesive taking up the same volume, the resulting softness or hand of the diaper 10 is improved compared to conventional designs. The expansion of the foamed adhesive 30 effectively forms a web-like structure of hot melt adhesive and gas that effectively adheres to the elastic strand 28 upon deposit on the elastic strand 28.

In another related example, foaming of the foamed adhesive 30 to a larger extent provides improved creep resistance for the same amount of add on adhesive weight. With reference to FIG. 14, test results are shown that prove the amount of creep resistance has been increased by the application of more foaming to the adhesive in operation. In this regard, FIG. 14 includes a graph 400 representing average creep in percent measured against add on adhesive weight in milligrams per meter of elastic strand 28. The creep measurements were taken after an extended aging period of 28 hours following bonding of the elastic strand 28 to a nonwoven substrate portion 18, and various data points (not shown) were plotted on the graph 400 for different levels of foaming (0% or liquid adhesive, 14%, 26%, and 34%) within the foamed adhesive 30. By "% foaming," the percentages shown in this Figure mean the percent of total volume expansion undergone by the foamed adhesive during and after deposit onto the elastic strand 28. Although the industry standard add on range is about 35-50 milligrams per meter, test results were provided both inside and outside this standard range so that trend lines 410a, 410b, 410c, 410d could be generated to illustrate the improvements for different levels of foaming the foamed adhesive 30.

As shown in the graph 400, the addition of more foaming to the foamed adhesive 30 significantly improved the resulting creep resistance over the extended sample aging period of 28 hours. To this end, the first trend line 410a for liquid adhesive (0% foam) shows a relatively high creep of about 25% for the industry standard add on range, while the second trend line 410b for 14% foaming drops the creep exhibited down toward 15% in the industry standard add on range. The third trend line 410c for 26% foaming further reduces the amount of creep exhibited in the test results, and the fourth trend line 410d for 34% foaming achieves an ideal amount of creep (e.g., less than 10%) within the industry standard add on range. Therefore, providing enough pressurized gas in the foamed adhesive 30 to result in at least 26% foaming or total volume expansion, or even more preferably, at least 34% foaming, provides a desirable level of creep resistance for most applications in the industry-standard add on range for adhesive weight per length of strand. It will be understood that the specific percentages of foaming and levels of creep resistance achieved may vary based on material differences for some adhesives in other embodiments. However, for all materials tested, increasing the amount of foaming that occurs by increasing the amount of pressurized gas entrained within the liquid hot melt adhesive will result in improved creep resistance for the same amount of add on weight. Thus, foaming the foamed adhesive 30 provides unexpected benefits in improving creep resistance while maintaining a high bond quality.

Furthermore, the expansion of the foamed adhesive 30 results in more rapid cooling of the outermost or external layers of adhesive material, and this reduces the likelihood of the foamed adhesive 30 burning through a substrate portion. More specifically, the foamed adhesive remains warm enough to form a reliable adhesive bond between elements of the diaper 10 while cooling enough to avoid burn-through on temperature sensitive substrates. Additionally, testing has also revealed that the use of foamed adhesive 30 reduces the pinch pressure that a pressure nip or pressure roller needs to apply to produce the high quality bond between the elastic strands 28 and the substrate portion 18. Thus, less forceful pressure nips may be utilized with the adhesive dispensing system 60 of this invention.

While the present invention has been illustrated by the description of specific embodiments thereof, and while the embodiments have been described in considerable detail, it is not intended to restrict or in any way limit the scope of the appended claims to such detail. The various features discussed herein may be used alone or in any combination. Additional advantages and modifications will readily appear to those skilled in the art. The invention in its broader aspects is therefore not limited to the specific details, representative apparatus and methods and illustrative examples shown and described. Accordingly, departures may be made from such details without departing from the scope of the general inventive concept.

## Claims

1. A method of manufacturing a disposable absorbent personal hygiene product including a stretched elastic strand and a first flat substrate portion, the method comprising:
moving the stretched elastic strand in a machine direction past a contact nozzle;
mixing a pressurized gas and hot melt adhesive to form a foamed adhesive;
contact dispensing the foamed adhesive at the contact nozzle by contacting the stretched elastic strand with the foamed adhesive;
expanding the foamed adhesive in volume during and after the contact dispensing onto the stretched elastic strand; and
securing the stretched elastic strand to the first flat substrate portion with the foamed adhesive.

2. The method of claim 1, wherein mixing the pressurized gas and hot melt adhesive further comprises:
mixing a sufficient quantity of pressurized gas with the hot melt adhesive to result in at least 14% total expansion in volume of the foamed adhesive contact dispensed onto the stretched elastic strand.

3. The method of claim 2, wherein mixing the pressurized gas and hot melt adhesive further comprises:
mixing a sufficient quantity of pressurized gas with the hot melt adhesive to result in at least 26% total expansion in volume of the foamed adhesive contact dispensed onto the stretched elastic strand.

4. The method of claim 3, wherein mixing the pressurized gas and hot melt adhesive further comprises:
mixing a sufficient quantity of pressurized gas with the hot melt adhesive to result in at least 34% total expansion in volume of the foamed adhesive contact dispensed onto the stretched elastic strand.

5. The method of claim 1, further comprising:
securing a second flat substrate portion to the stretched elastic strand and to the first flat substrate portion with the foamed adhesive to enclose the stretched elastic strand.

6. The method of claim 5, wherein the first and second flat substrate portions include separate first and second substrates, and the method further comprises:
enclosing the stretched elastic strand between the first and second substrates.

7. The method of claim 5, wherein the first and second flat substrate portions include a single flat substrate, and the method further comprises:
folding a first section of the single flat substrate over the stretched elastic strand and a second section of the single flat substrate; and
enclosing the stretched elastic strand between the first and second sections of the single flat substrate.

8. The method of claim 1, wherein the disposable absorbent personal hygiene product includes a plurality of stretched elastic strands, and the method comprises:
moving the plurality of stretched elastic strands in a machine direction past the contact nozzle;
contact dispensing the foamed adhesive at the contact nozzle by contacting the plurality of stretched elastic strands with the foamed adhesive, such that the foamed adhesive expands in volume on the stretched elastic strands; and
securing the plurality of stretched elastic strands to the first flat substrate portion with the foamed adhesive.

9. The method of claim 8, further comprising:
discharging air at the foamed adhesive on the plurality of stretched elastic strands at a location downstream from the contact nozzle in the machine direction so that the foamed adhesive further spreads around on the periphery of the plurality of stretched elastic strands.

10. The method of claim 1, wherein contact dispensing the foamed adhesive further comprises:
supplying the foamed adhesive to the contact nozzle in a discontinuous manner such that the foamed adhesive is discharged in a discontinuous manner into contact with the stretched elastic strand, wherein the foamed adhesive forms localized masses configured to become discrete bond points when securing the stretched elastic strand to the first flat substrate portion.

11. The method of claim 10, wherein supplying the foamed adhesive to the contact nozzle in a discontinuous manner further comprises:
repeatedly turning on and off a supply of the foamed adhesive, thereby producing coating portions on the stretched elastic strand separated by void portions having less foamed adhesive than the coating portions.

12. The method of claim 11, wherein repeatedly turning on and off the supply of the foamed adhesive further comprises:
controlling the supply of the foamed adhesive to contact dispense a first volume of foamed adhesive per unit length of the stretched elastic strand onto a first portion of the stretched elastic strand;
controlling the supply of the foamed adhesive to contact dispense a second volume of foamed adhesive per unit length of the stretched elastic strand onto a second portion of the stretched elastic strand located downstream from the first portion relative to the machine direction; and
controlling the supply of the foamed adhesive to contact dispense a third volume of foamed adhesive per unit length of the stretched elastic strand onto a third portion of the stretched elastic strand located downstream from the second portion relative to the machine direction,
wherein the second volume per unit length is less than the first volume per unit length and less than the third volume per unit length, and wherein the first and third portions of the stretched elastic strand define opposing ends of the stretched elastic strand when adhered to the first flat substrate portion.

13. The method of claim 1, wherein mixing the pressurized gas and hot melt adhesive further comprises:
increasing an amount of pressurized gas that is mixed with a predetermined volume of hot melt adhesive to increase the amount of foaming that the foamed adhesive will undergo following contact dispensing, thereby increasing creep resistance of the stretched elastic strand after securing to the first flat substrate portion.

14. The method of claim 1, wherein the contact nozzle includes a notch for receiving the stretched elastic strand moving in the machine direction, and the method further comprises:
spreading the foamed adhesive around on a periphery of the stretched elastic strand by wiping the foamed adhesive onto the periphery with the notch.

15. The method of claim 14, further comprising:
discharging air at the foamed adhesive on the stretched elastic strand at a location downstream from the contact nozzle in the machine direction so that the foamed adhesive further spreads around on the periphery of the stretched elastic strand.

16. The method of claim 1, wherein the contact nozzle includes a converging slit communicating with a strand slot configured to receive the stretched elastic strand moving in the machine direction, and contact dispensing the foamed adhesive further comprises:
supplying the foamed adhesive through the converging slit to increase or maintain a pressure of the foamed adhesive prior to contact dispensing at the strand slot, thereby limiting any foaming of the foamed adhesive before contact dispensing onto the stretched elastic strand.

## Patentansprüche

1. Verfahren zum Herstellen eines absorbierenden Personenhygieneeinwegprodukts, umfassend einen gedehnten elastischen Strang und einen ersten flachen Substratabschnitt, wobei das Verfahren aufweist:
Bewegen des gedehnten elastischen Strangs in eine Maschinenrichtung entlang einer Kontaktdüse;
Mischen eines Druckgases und eines Heißschmelzklebstoffs zum Bilden eines geschäumten Klebstoffs;
Kontaktabgeben des geschäumten Klebstoffs bei der Kontaktdüse durch Kontaktieren des gedehnten elastischen Strangs mit dem geschäumten Klebstoff;
Expandieren des geschäumten Klebstoffs im Volumen während und nach dem Kontaktabgegeben auf den gedehnten elastischen Strang und;
Verbinden des gedehnten elastischen Strangs mit dem ersten flachen Substratabschnitt mit dem geschäumten Klebstoff.

2. Verfahren nach Anspruch 1, wobei Mischen des Druckgases und des Heißschmelzklebstoffs ferner aufweist:
Mischen einer ausreichenden Menge von Druckgas mit dem Heißschmelzklebstoff zum Erhalten von wenigstens 14 % Gesamtexpansion im Volumen des geschäumten Klebstoffs, der auf den gedehnten elastischen Faden Kontakt abgegeben wurde.

3. Verfahren nach Anspruch 2,
wobei Mischen des Druckgases und des Heißschmelzklebstoffs ferner aufweist:
Mischen einer ausreichenden Menge von Druckgas mit dem Heißschmelzklebstoff zum Erhalten von wenigstens 26 % Gesamtexpansion im Volumen des geschäumten Klebstoffs, der auf den gedehnten elastischen Faden Kontakt abgegeben wurde.

4. Verfahren nach Anspruch 3,
wobei Mischen des Druckgases und des Heißschmelzklebstoffs ferner aufweist:
Mischen einer ausreichenden Menge von Druckgas mit dem Heißschmelzklebstoff zum Erhalten von wenigstens 34 % Gesamtexpansion im Volumen des geschäumten Klebstoffs, der auf den gedehnten elastischen Faden Kontakt abgegeben wurde.

5. Verfahren nach Anspruch 1, ferner aufweisend:
Verbinden eines zweiten flachen Substratabschnitts mit dem gedehnten elastischen Strang und dem ersten flachen Substratabschnitt mit dem geschäumten Klebstoff zum Einschließen des gedehnten elastischen Strangs.

6. Verfahren nach Anspruch 5, wobei die ersten und zweiten flachen Substratabschnitte separate erste und zweite Substrate umfassen, und wobei das Verfahren ferner aufweist:
Einschließen des gedehnten elastischen Strangs zwischen den ersten und zweiten Substraten.

7. Verfahren nach Anspruch 5, wobei die ersten und zweiten flachen Substratabschnitte ein einzelnes flaches Substrat umfassen, und das Verfahren ferner aufweist:
Falten eines ersten Teils des einzelnen flachen Substrats über den gedehnten elastischen Strang und einen zweiten Teil des einzelnen flachen Substrats; und
Einschließen des gedehnten elastischen Strangs zwischen den ersten und zweiten Teilen des einzelnen flachen Substrats.

8. Verfahren nach Anspruch 1, wobei das absorbierende Personenhygieneeinwegprodukt eine Mehrzahl an gedehnten elastischen Strängen umfasst, und das Verfahren aufweist:
Bewegen der Mehrzahl gedehnter elastischer Stränge in eine Maschinenrichtung entlang der Kontaktdüse;
Kontaktabgeben des geschäumten Klebstoffs bei der Kontaktdüse durch Kontaktieren der Mehrzahl gedehnter elastischer Stränge mit dem geschäumten Klebstoff, so dass der geschäumte Klebstoff im Volumen auf den gedehnten elastischen Strängen expandiert; und
Verbinden der Mehrzahl gedehnter elastischer Stränge mit dem ersten flachen Substratabschnitt mit dem geschäumten Klebstoff.

9. Verfahren nach Anspruch 8, ferner aufweisend:
Abgeben von Luft auf den geschäumten Klebstoff auf der Mehrzahl gedehnter elastischer Stränge an einem Ort stromabwärts der Kontaktdüse in der Maschinenrichtung, so dass sich der geschäumte Klebstoff weiter um den Umfang der Mehrzahl elastischer Stränge verteilt.

10. Verfahren nach Anspruch 1, wobei Kontaktabgeben des geschäumten Klebstoffs ferner aufweist:
Zuführen des geschäumten Klebstoffs in einer diskontinuierlichen Weise zu der Kontaktdüse, so dass der geschäumte Klebstoff in einer diskontinuierlichen Weise in Kontakt mit dem gedehnten elastischen Strang abgegeben wird, wobei der geschäumte Klebstoff lokalisierte Massen bildet, die dazu ausgebildet sind diskrete Verbindungspunkte zu werden, wenn der gedehnte elastische Strang mit dem ersten flachen Substratabschnitt verbunden wird.

11. Verfahren nach Anspruch 10, wobei Zuführen des geschäumten Klebstoffs in einer diskontinuierlichen Weise zu der Kontaktdüse ferner aufweist:
wiederholtes Ein- und Ausschalten einer Zuführung des geschäumten Klebstoffs, wodurch Beschichtungsabschnitte auf dem gedehnten elastischen Strang erzeugt werden, die durch Leer-Abschnitte getrennt sind, die weniger geschäumten Klebstoff als die Beschichtungsabschnitte aufweisen.

12. Verfahren nach Anspruch 11, wobei das wiederholte Ein- und Ausschalten der Zuführung des geschäumten Klebstoffs ferner aufweist:
Steuern der Zuführung des geschäumten Klebstoffs zum Kontaktabgeben eines ersten Volumens geschäumten Klebstoffs pro Längeneinheit des gedehnten elastischen Strangs auf einen ersten Abschnitt des gedehnten elastischen Strangs;
Steuern der Zuführung des geschäumten Klebstoffs zum Kontaktabgeben eines zweiten Volumens geschäumten Klebstoffs pro Längeneinheit des gedehnten elastischen Strangs auf einen zweiten Abschnitt des gedehnten elastischen Strangs, der stromabwärts des ersten Abschnitts bezogen auf die Maschinenrichtung angeordnet ist; und
Steuern der Zuführung des geschäumten Klebstoffs zum Kontaktabgeben eines dritten Volumens geschäumten Klebstoffs pro Längeneinheit des gedehnten elastischen Strangs auf einen dritten Abschnitt des gedehnten elastischen Strangs, der stromabwärts des zweiten Abschnitts bezogen auf die Maschinenrichtung angeordnet ist,
wobei das zweite Volumen pro Längeneinheit geringer ist als das erste Volumen pro Längeneinheit und geringer ist als das dritte Volumen pro Längeneinheit, und wobei die ersten und zweiten Abschnitte des gedehnten elastischen Strangs gegenüberliegende Enden des gedehnten elastischen Strangs definieren, wenn dieser mit dem ersten flachen Substratabschnitt verklebt wird.

13. Verfahren nach Anspruch 1, wobei Mischen des Druckgases und des Heißschmelzklebstoffs ferner aufweist:
Erhöhen einer Menge an Druckgas, die mit einem vorbestimmten Volumen des Heißschmelzklebstoffs gemischt wird zum Erhöhen der Schäumungsmenge, die der geschäumte Klebstoff folgend dem Kontaktabgeben erfährt, und dadurch Erhöhen des Kriechwiderstands des gedehnten elastischen Strangs nach dem Verbinden mit dem ersten flachen Substratabschnitt.

14. Verfahren nach Anspruch 1, wobei die Kontaktdüse eine Kerbe zum Empfangen des gedehnten elastischen Strangs, der in Maschinenrichtung bewegt wird, umfasst, und das Verfahren ferner aufweist:
Verteilen des geschäumten Klebstoffs um einen Umfang des gedehnten elastischen Strangs durch Wischen des geschäumten Klebstoffs auf dem Umfang mittels der Kerbe.

15. Verfahren nach Anspruch 14, ferner aufweisend:
Abgeben von Luft auf den geschäumten Klebstoff auf den gedehnten elastischen Strang an einem Ort stromabwärts der Kontaktdüse in Maschinenrichtung, so dass sich der geschäumte Klebstoff weiter um den Umfang des gedehnten elastischen Strangs verteilt.

16. Verfahren nach Anspruch 1, wobei die Kontaktdüse einen zusammenlaufenden Schlitz umfasst, der mit einem Strangschlitz in Verbindung steht, der dazu eingerichtet ist den gedehnten elastischen Strang zu empfangen, der sich in Maschinenrichtung bewegt, und wobei das Kontaktabgeben des geschäumten Klebstoffs ferner aufweist:
Zuführen des geschäumten Klebstoffs durch den zusammenlaufenden Schlitz zum Erhöhen oder Aufrechterhalten eines Drucks des geschäumten Klebstoffs vor dem Kontaktabgeben an dem Strangschlitz, und dadurch Begrenzen jeglichen Schäumens des geschäumten Klebstoffs vor Kontaktabgeben auf den gedehnten elastischen Strang.

## Revendications

1. Procédé de fabrication d'un produit d'hygiène personnelle absorbant jetable comprenant un fil élastique étiré et une première partie de substrat plat, le procédé comprenant :
le déplacement du fil élastique étiré dans un sens machine au-delà d'une buse de contact ;
le mélange d'un gaz pressurisé et d'un adhésif thermofusible pour former un adhésif expansé ;
la distribution par contact de l'adhésif expansé au niveau de la buse de contact par mise en contact du fil élastique étiré et de l'adhésif expansé ;
l'expansion de l'adhésif expansé en volume pendant et après la distribution par contact sur le fil élastique étiré ; et
la fixation du fil élastique étiré à la première partie de substrat plat avec l'adhésif expansé.

2. Procédé selon la revendication 1, dans lequel le mélange du gaz pressurisé et de l'adhésif thermofusible comprend en outre :
le mélange d'une quantité suffisante de gaz pressurisé avec l'adhésif thermofusible pour résulter en une expansion totale d'au moins 14 % en volume de l'adhésif expansé distribué par contact sur le fil élastique étiré.

3. Procédé selon la revendication 2, dans lequel le mélange du gaz pressurisé et de l'adhésif thermofusible comprend en outre :
le mélange d'une quantité suffisante de gaz pressurisé avec l'adhésif thermofusible pour résulter en une expansion totale d'au moins 26 % en volume de l'adhésif expansé distribué par contact sur le fil élastique étiré.

4. Procédé selon la revendication 3, dans lequel le mélange du gaz pressurisé et de l'adhésif thermofusible comprend en outre :
le mélange d'une quantité suffisante de gaz pressurisé avec l'adhésif thermofusible pour résulter en une expansion totale d'au moins 34 % % en volume de l'adhésif expansé distribué par contact sur le fil élastique étiré.

5. Procédé selon la revendication 1, comprenant en outre :
la fixation d'une deuxième partie de substrat plat au fil élastique étiré et à la première partie de substrat plat avec l'adhésif expansé pour enfermer le fil élastique étiré.

6. Procédé selon la revendication 5, dans lequel les première et deuxième parties de substrat plat comprennent des premier et deuxième substrats séparés, et le procédé comprend en outre :
le fait d'enfermer le fil élastique étiré entre les premier et deuxième substrats.

7. Procédé selon la revendication 5, dans lequel les première et deuxième parties de substrat plat comprennent un substrat plat unique, et le procédé comprend en outre :
le pliage d'une première section du substrat plat unique sur le fil élastique étiré et une deuxième section du substrat plat unique ; et
le fait d'enfermer le fil élastique étiré entre les première et deuxième parties du substrat plat unique.

8. Procédé selon la revendication 1, dans lequel le produit d'hygiène personnelle absorbant jetable comprend une pluralité de fils élastiques étirés, et le procédé comprend :
le déplacement de la pluralité de fils élastiques étirés dans un sens machine au-delà de la buse de contact ;
la distribution par contact de l'adhésif expansé au niveau de la buse de contact par mise en contact de la pluralité de fils élastiques étirés et de l'adhésif expansé, de telle sorte que l'adhésif expansé se dilate en volume sur les fils élastiques étirés ; et
la fixation de la pluralité de fils élastiques étirés à la première partie de substrat plat avec l'adhésif expansé.

9. Procédé selon la revendication 8, comprenant en outre :
la décharge d'air au niveau de l'adhésif expansé sur la pluralité de fils élastiques étirés à un emplacement en aval de la buse de contact dans le sens machine de telle sorte que l'adhésif expansé se répande davantage autour de la périphérie sur la pluralité de fils élastiques étirés.

10. Procédé selon la revendication 1, dans lequel la distribution par contact de l'adhésif expansé comprend en outre :
l'alimentation discontinue en adhésif expansé à la buse de contact de telle sorte que l'adhésif expansé est déchargé de manière discontinue en contact avec le fil élastique étiré, dans lequel l'adhésif expansé forme des masses localisées conçues pour devenir des points de liaison discrets lors de la fixation du fil élastique étiré à la première partie de substrat plat.

11. Procédé selon la revendication 10, dans lequel l'alimentation discontinue en adhésif expansé à la buse de contact comprend en outre :
le fait d'activer et de désactiver plusieurs fois une alimentation en adhésif expansé, produisant ainsi des parties de revêtement sur le fil élastique étiré séparées par des parties vides ayant moins d'adhésif expansé que les parties de revêtement.

12. Procédé selon la revendication 11, dans lequel le fait d'activer et de désactiver à répétition une alimentation en adhésif expansé comprend en outre :
la commande de l'alimentation en adhésif expansé pour distribuer par contact un premier volume d'adhésif expansé par longueur unitaire du fil élastique étiré sur une première partie du fil élastique étiré ;
la commande de l'alimentation en adhésif expansé pour distribuer par contact un deuxième volume d'adhésif expansé par longueur unitaire du fil élastique étiré sur une deuxième partie du fil élastique étiré située en aval de la première partie relativement au sens machine ; et
la commande de l'alimentation en adhésif expansé pour distribuer par contact un troisième volume d'adhésif expansé par longueur unitaire du fil élastique étiré sur une troisième partie du fil élastique étiré situé en aval de la deuxième partie relativement au sens machine ;
dans lequel le deuxième volume par longueur unitaire est inférieur au premier volume par longueur unitaire et inférieur au troisième volume par longueur unitaire, et dans lequel les première et troisième parties du fil élastique étiré définissent des extrémités opposées du fil élastique étiré lorsque celui-ci adhère à la première partie du substrat plat.

13. Procédé selon la revendication 1, dans lequel le mélange du gaz pressurisé et de l'adhésif thermofusible comprend en outre :
l'augmentation d'une quantité de gaz pressurisé qui est mélangée avec un volume prédéterminé d'adhésif thermofusible pour faire augmenter la quantité d'expansion que l'adhésif expansé subira après la distribution par contact, permettant ainsi d'augmenter la résistance au fluage du fil élastique étiré après la fixation à la première partie de substrat plat.

14. Procédé selon la revendication 1, dans lequel la buse de contact comprend une encoche pour recevoir le fil élastique étiré se déplaçant dans le sens machine, et le procédé comprend en outre :
la diffusion de l'adhésif expansé autour d'une périphérie du fil élastique étiré en déposant l'adhésif expansé sur la périphérie avec l'encoche.

15. Procédé selon la revendication 14, comprenant en outre :
la décharge d'air au niveau de l'adhésif expansé sur le fil élastique étiré à un emplacement en aval de la buse de contact dans le sens machine de telle sorte que l'adhésif expansé se répande davantage autour de la périphérie du fil élastique étiré.

16. Procédé selon la revendication 1, dans lequel la buse de contact comprend une fente convergente communiquant avec une fente de fil conçue pour recevoir le fil élastique étiré se déplaçant dans le sens machine, et la distribution par contact de l'adhésif expansé comprend en outre :
l'alimentation en adhésif expansé à travers la fente convergente pour augmenter ou maintenir une pression de l'adhésif expansé avant la distribution par contact au niveau de la fente de fil, limitant ainsi toute expansion de l'adhésif expansé avant la distribution par contact sur le fil élastique étiré.
